# EUROPEAN PATENT SPECIFICATION

(11) **EP 2 524 698 B1**
(45) Date of publication and mention of the grant of the patent: **21.11.2018**
(21) Application number: 11732894.8
(22) Date of filing: 12.01.2011
(51) Int. Cl.: A61K 38/28, A61K 47/42, A61P 3/10, A61K 9/00

(54) **INSULIN PREPARATION**
INSULINHERSTELLUNG
PRÉPARATION D'INSULINE

(30) Priority: 16.07.2010 JP 2010161767; 14.01.2010 JP 2010005540
(43) Date of publication of application: 21.11.2012
(73) Proprietor: The Doshisha, Kyoto-shi, Kyoto 602-8580 (JP)
(72) Inventor: SHIBATA, Nobuhito, Kyoto 610-0395 (JP); NISHIMURA, Asako, Kyoto 610-0395 (JP)
(74) Representative: J A Kemp
(86) International application number: PCT/JP2011/050371
(87) International publication number: WO 2011/087024

(56) References cited:
- WO-A1-2009/072556
- WO-A1-2009/155334
- WO-A2-2006/073889
- JP-A- 2005 515 796
- US-A1- 2004 087 013
- BRANCO M C ET AL: "Self-assembling materials for therapeutic delivery", ACTA BIOMATERIALIA, ELSEVIER, AMSTERDAM, NL, vol. 5, no. 3, 1 March 2009 (2009-03-01), pages 817-831, XP025990679, ISSN: 1742-7061, DOI: 10.1016/J.ACTBIO.2008.09.018 [retrieved on 2008-10-10]
- YAMAOKA H ET AL.: 'Cartilage tissue engineering using human auricular chondrocytes embedded in different hydrogel materials.' J BIOMED MATER RES A vol. 78, no. 1, 2006, pages 1 - 11, XP008163037
- HIDEKI SHIGEMATSU ET AL.: 'Kotsu Keisei Inshi BMP-2 no Joho Device to shite no PuraMatrix no Koka' JAPANESE JOURNAL OF TRANSPLANTATION vol. 43, no. 2, 2008, page 152, XP008163041

### [SEQUENCE LISTINGS]

### Sequence Listings

### SEQUENCE LISTING

<110> The Doshisha
<120> Insulin perparation
<130> N.117408
<140> EP 11732894.8
   <141> 2011-01-12
<150> JP 2010-161767
   <151> 2010-07-16
<150> JP 2010-005540
   <151> 2010-01-14
<160> 1
<210> 1
   <211> 16
   <212> PRT
   <213> Artificial
<220>
   <223> designed peptide
<220>
   <221> MOD_RES
   <222> (1)..(1)
   <223> ACETYLATION
<400> 1
   Arg Ala Asp Ala Arg Ala Asp Ala Arg Ala Asp Ala Arg Ala Asp Ala
   1               5                   10                  15

## Description

### TECHNICAL FIELD

The present invention relates to an insulin preparation.

### BACKGROUND ART

According to the World Health Organization (WHO), there were at least 171 million diabetes patients worldwide as of 2006. The number of patients is increasing rapidly, and their number is predicted to double by 2030. In Japan, the number of diabetes patients has risen from about 30,000 to about 7 million over the past 40 years, and the number further increases to 20 million when borderline diabetes (pre-diabetes) patients are included.

At present, various insulin preparations for subcutaneous injection are commercially available. These preparations can be classified into ultra-fast-acting types, fast-acting types, intermediate types, mixed types and long-acting types according to the speed at which they demonstrate their effects.

Ultra-fast-acting types consist of water-soluble preparations in the form of human insulin analogues such as Insulin Aspart or Insulin Lispro. These preparations enhance the diffusibility and absorptivity of insulin in tissue following subcutaneous injection by avoiding the formation of insulin hexamers.

Fast-acting types primarily consist of water-soluble preparations using conventional semi-synthetic insulin. On the other hand, intermediate type and mixed type insulin preparations which use human insulin, are designed so as to inhibit decomposition from hexamers thereof, and are used in the form of a white suspension. In addition, long-acting type preparations use water-soluble preparations using human insulin analogues such as Insulin Glargine or Insulin Detemir that inhibit decomposition from insulin hexamers.

In the clinical setting, these insulin preparations are currently used alone or concomitantly corresponding to basal insulin secretion levels and fluctuation patterns.

However, as is observed in cases of intermediate and mixed preparations in particular, cases have been reported in which blood glucose levels are not adequately controlled in cases in which preparations in the form of white suspensions are not adequately mixed by inverting prior to use. Thus, the current treatment of diabetes using these preparations is such that the properties of the insulin per se as well as the properties of the preparation have a considerable influence on therapeutic efficacy. In addition, insulin analogues are associated with the shortcoming of being more expensive than prototype insulin.

Self-assembling peptides have the characteristic of forming a self-associated form containing a large number of peptide molecules arranged in an orderly manner according to the amino acid sequence thereof. These self-assembling peptides have attracted attention in recent years for use as novel materials based on their physical, chemical and biological properties.

Self-assembling peptides have a structure in which charged hydrophilic amino acids and electrically neutral hydrophobic amino acids are alternately arranged so that positive charge and negative charge are alternately distributed, and adopt a β structure at physiological pH and acidity.

Acidic amino acids selected from aspartic acid and glutamic acid as well as basic amino acids selected from arginine, lysine, histidine and ornithine can be used as hydrophilic amino acids. Alanine, valine, leucine, isoleucine, methionine, phenylalanine, tyrosine, tryptophan, serine, threonine or glycine can be used as hydrophobic amino acids.

Hydrogels containing self-assembling peptides are biodegradable, and are suitable for cell survival and proliferation since degradation products thereof do not have a detrimental effect on tissue and have high bioabsorptivity.

Holmes, et al. described that a hydrogel containing self-assembling peptide is useful as a sustained-release carrier of insulin and other protein-based preparations (Patent Document 1). However, carriers for sustained release of a specific drug have not been actually produced, and drug selection and drug release time have yet to be optimized.

Patent document 2 discloses a therapeutic composition in which human PDGF is bound directly to peptides that self-assemble into a biologically compatible gel.

Patent document 3 discloses a wound healing/skin reconstruction material containing a peptide, wherein the peptide is an amphiphilic peptide having 8 to 200 amino acid residues in which the hydrophilic amino acids and hydrophobic amino acids are alternately linked to one another, and is a self-assembled peptide showing a stable β-sheet structure in an aqueous solution in the presence of a monovalent ion.

Non-patent document 1 provides an overview of self-assembling molecules, their resulting structures and their use in therapeutic delivery.

Non-patent document 2 discloses a comparison of various hydrogel materials originating from animals, plants or synthetic peptides.

[Patent Document 1] U.S. Patent No. 7098028
[Patent Document 2] WO 2006/073889
[Patent Document 3] WO 2009/072556

[Non-patent document 1] Branco et al, (2009) Acta Biomaterialia, 5, 817-831
[Non-patent document 2] Yamaoka et al (2006) J Biomed Mater Res A, 78, 1-11

### DISCLOSURE OF THE INVENTION

### [Problems to be Solved by the Invention]

The present invention provides a novel insulin preparation capable of constant release of water-soluble insulin and which is able to control the amount of insulin released, by using a single type of insulin.

### [Means for Solving the Problems]

The present invention relates to an insulin preparation that contains a self-assembling peptide.

### [Effects of the Invention]

The insulin preparation of the present invention enables constant release of water-soluble insulin and is able to control the amount of insulin released, by using a single type of insulin.

### BRIEF DESCRIPTION OF THE DRAWINGS

FIG. 1 indicates percentage changes in plasma glucose concentrations after subcutaneous administration of PM preparation formulas 1 to 3 and a control to rats (N=6, insulin concentration: 10 IU/kg (final concentration), injection volume: 200 µL/kg).
FIG. 2 indicates percentage changes in plasma glucose levels after subcutaneous administration of PM preparation formulas 2 and 4 and a control to rats (N=4, insulin concentration: 10 IU/kg (final concentration), injection volume: 200 µL/kg).
FIG. 3 indicates percentage changes in plasma glucose concentrations after subcutaneous administration of PM preparation formulas 4 to 10 and a control to rats (N=6, insulin concentration: 10 IU/kg (final concentration), injection volume: 200 µL/kg).
FIG. 4 indicates percentage changes in plasma glucose concentrations after subcutaneous administration of PM preparation formulas 4 to 10 and a control to rats (N=6, insulin concentration: 10 IU/kg (final concentration), injection volume: 200 µL/kg).
FIG. 5 indicates percentage changes in plasma glucose concentrations after subcutaneous administration of PM preparation formulas 4 to 10 and a control to rats (N=6, insulin concentration: 10 IU/kg (final concentration), injection volume: 200 µL/kg).
FIG. 6 indicates a comparison of RPA values between the PM preparation and Humulin™R.

### BEST MODE FOR CARRYING OUT THE INVENTION

The inventors of the present invention found that the release of water-soluble insulin can be made to be constant and the amount of insulin released can be controlled, by using a single type of insulin by applying a self-assembling peptide hydrogel that is used as a scaffold of cell culture to DDS, thereby leading to completion of the present invention.

Namely, the present invention is as indicated below.
[1] A long-acting insulin preparation for use in a method of treating diabetes, comprising insulin, a self-assembling peptide of SEQ ID NO:1, and a hydrogen and a chloride ion, wherein the concentration of the self-assembling peptide of SEQ ID NO: 1 is 1.0 to 2.0% (w/v).
[2] An ultra-fast insulin preparation for use in a method of treating diabetes comprising insulin, a self-assembling peptide of SEQ ID NO: 1, and a hydrogen and chloride ion, wherein the concentration of the self-assembling peptide of SEQ ID NO: 1 is 0.1 to 0.25% (w/v).
[3] The insulin preparation according to [1] or [2], wherein the insulin is wild-type insulin.

The self-assembling peptide in the present invention is peptide RAD16 having the sequence (Ac-(RADA)₄-CONH₂) (SEQ ID NO:1). RAD16 is commercially available from 3-D Matrix Ltd. in the form of a 1% (w/v) aqueous solution of PuraMatrix™. PuraMatrix™ also contains hydrogen ion and chloride ion in addition to 1% (w/v) of peptide having an (Ac-(RADA)₄-CONH₂) sequence (SEQ ID NO:1).

In addition to eliminating the risk of viral and other infections in comparison with conventional materials of biological origin since the self-assembling peptide of the present invention can be produced synthetically, there is also no need for concerning over inflammation and the like since the peptide per se is bioabsorbable.

Any arbitrary commercially available insulin for subcutaneous injection can be used for the insulin used in the present invention. Wild-type insulin in the manner of semi-synthetic human insulin or insulin analogues in the manner of Insulin Aspart, Insulin Lispro, Insulin Glargine or Insulin Detemir can be used preferably. Wild-type insulin is more preferable.

### [Example 1]

Although the following provides a more detailed explanation of the insulin preparation of the present invention through examples thereof, the present invention is not limited thereto provided they do not deviate from the gist or application range thereof.

### Production of 1% PM and 3% PM Using PuraMatrix™ (PM)

PM was dissolved in ultrapure water (Milli-Q) to prepare 1% (w/v) and 3% (w/v) solutions thereof. Furthermore, bovine pancreatic insulin (29 IU/g) was used for the insulin.

Insulin preparations were prepared according to the formulas indicated below.

### Formula 1: 0.2% (w/v) PM-Insulin Preparation

| | |
|---|---|
| Insulin | 5 mg |
| 0.01 N HCl | 580 µL |
| 1% (w/v) PM | 580 µL |
| Milli-Q | 1740 µL |
| Total volume | 2900 µL |

### Formula 2: 0.5% (w/v) PM-Insulin Preparation

| | |
|---|---|
| Insulin | 5 mg |
| 0.01 N HCl | 580 µL |
| 1% (w/v) PM | 1450 µL |
| Milli-Q | 870 µL |
| Total volume | 2900 µL |

### Formula 3: 0.8% (w/v) PM-Insulin Preparation

| | |
|---|---|
| Insulin | 5 mg |
| 0.01 N HCl | 580 µL |
| 1% (w/v) PM | 2320 µL |
| Milli-Q | 0 µL |
| Total volume | 2900 µL |

### Formula 4: 1.5% (w/v) PM-Insulin Preparation

| | |
|---|---|
| Insulin | 5 mg |
| 0.01 N HCl | 580 µL |
| 3% (w/v) PM | 1450 µL |
| Milli-Q | 870 µL |
| Total volume | 2900 µL |

### Preparation of Control and Positive Control

PM only at 1% (w/v) was administered as a control. A preparation containing semi-synthetic human insulin that is currently commercially available (fast-acting water-soluble preparation, Humulin™R) was used as a positive control.

### Animal Study

Wistar male rats (age 9 to 10 weeks) fasted for 12 to 16 hours were anesthetized with urethane (1 g/kg), followed by subcutaneous administration of each of the insulin preparations (Formulas 1 to 4), control and positive control into the abdomens of the animals while under anesthesia. The insulin dose was 10 IU/kg for all animals (final concentration). 100 µL of whole blood were collected from the jugular vein into heparinized centrifuge tubes before subcutaneous administration and at 30 minutes and 1, 2, 3, 4, 5, 6, 9, 12 and 24 hours after subcutaneous administration followed by separation of plasma.

Glucose concentration in the plasma was measured by colorimetry using a commercially available measurement kit. The plasma glucose level prior to administration of insulin preparation was used as a baseline (100%), and changes in plasma glucose levels were indicated as a percentage (%) relative to that baseline.

### Study up to 6 Hours after Subcutaneous Administration

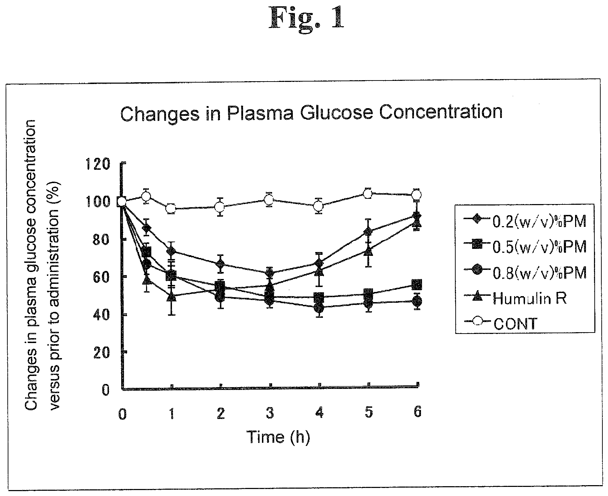

Decreases in plasma glucose levels became larger as PM concentration increased. Decreases in plasma glucose concentrations were observed for at least 6 hours in the case of the 0.5% (w/v) and 0.8% (w/v) PM formulas. In addition, the maximum decrease in plasma glucose concentration was roughly equal to that of fast-acting Humulin™R currently used in the clinical setting. In addition, PM per se was not observed to demonstrate hypoglycemic action (FIG. 1).

### Study up to 24 Hours after Subcutaneous Administration

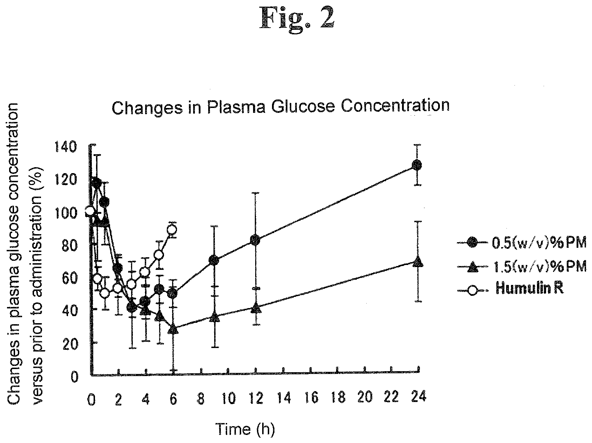

Decreases in plasma glucose concentrations were observed for at least 24 hours in the case of a PM concentration of 0.5% (w/v). Decreases in plasma glucose concentrations were observed beyond 24 hours when the PM concentration was 1.5% (w/v) (FIG. 2).

### [Example 2]

### Production of Insulin Preparations Using 1% (w/v)

PuraMatrix™ (PM)

PM was dissolved in ultrapure water (Milli-Q) to prepare 1% (w/v) and 3% (w/v) solutions thereof. Furthermore, bovine pancreatic insulin (29 IU/g) was used for the insulin.

An insulin preparation was prepared according to the formula indicated below.

### Formula 1: 0.1% (w/v) PM-Insulin Preparation

| | |
|---|---|
| Insulin | 5 mg |
| 0.01 N HCl | 580 µL |
| 1% (w/v) PM | 290 µL |
| Milli-Q | 2030 µL |
| Total volume | 2900 µL |

Insulation preparations were also prepared according to formulas indicated below.

### Formula 2: 0.25% (w/v) PM-Insulin Preparation

| | |
|---|---|
| Insulin | 5 mg |
| 0.01 N HCl | 580 µL |
| 1% (w/v) PM | 725 µL |
| Milli-Q | 1595 µL |
| Total volume | 2900 µL |

### Formula 3: 0.5% (w/v) PM-Insulin Preparation

| | |
|---|---|
| Insulin | 5 mg |
| 0.01 N HCl | 580 µL |
| 1% (w/v) PM | 1450 µL |
| Milli-Q | 870 µL |
| Total volume | 2900 µL |

### Formula 4: 1.0% (w/v) PM-Insulin Preparation

| | |
|---|---|
| Insulin | 5 mg |
| 0.01 N HCl | 580 µL |
| 3% (w/v) PM | 967 µL |
| Milli-Q | 1353 µL |
| Total volume | 2900 µL |

### Formula 5: 1.5% (w/v) PM-Insulin Preparation

| | |
|---|---|
| Insulin | 5 mg |
| 0.01 N HCl | 580 µL |
| 3% (w/v) PM | 1450 µL |
| Milli-Q | 870 µL |
| Total volume | 2900 µL |

### Formula 6: 2.0% (w/v) PM-Insulin Preparation

| | |
|---|---|
| Insulin | 5 mg |
| 0.01 N HCl | 580 µL |
| 3% (w/v) PM | 1933 µL |
| Milli-Q | 387 µL |
| Total volume | 2900 µL |

### Positive Control Preparation

A preparation containing semi-synthetic human insulin that is currently commercially available (fast-acting water-soluble preparation, Humulin™R) was used for the positive control.

### Animal Study

Wistar male rats (age 9 to 10 weeks) fasted for 12 to 16 hours were anesthetized with urethane (1 g/kg), followed by subcutaneous administration of each of the insulin preparations (Formulas 1 to 6), control and positive control into the abdomens of the animals while under anesthesia. The insulin dose was 10 IU/kg for all animals (final concentration). 100 µL of whole blood were collected from the jugular vein into heparinized centrifuge tubes before subcutaneous administration and at 30 minutes and 1, 2, 3, 4, 5, 6, 9, 12 and 24 hours after subcutaneous administration followed by separation of plasma.

Glucose concentration in the plasma was measured by colorimetry using a commercially available measurement kit. The plasma glucose level prior to administration of insulin preparation was used as a baseline (100%), and changes in plasma glucose levels were indicated as a percentage (%) relative to that baseline.

### Study up to 6 Hours after Subcutaneous Administration

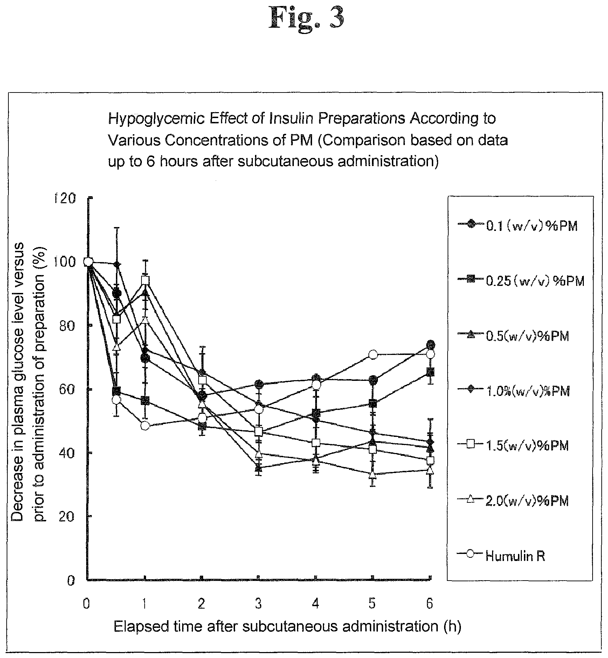

Decreases in plasma glucose levels became larger as PM concentration increased. Decreases in plasma glucose concentrations were observed for at least 6 hours in the case of the 0.5% (w/v), 1.0% (w/v), 1.5% (w/v) and 2.0% (w/v) PM formulas. In addition, the maximum decrease in plasma glucose concentration was roughly equal to that of fast-acting Humulin™R currently used in the clinical setting (FIG. 3).

### Study up to 12 Hours after Subcutaneous Administration

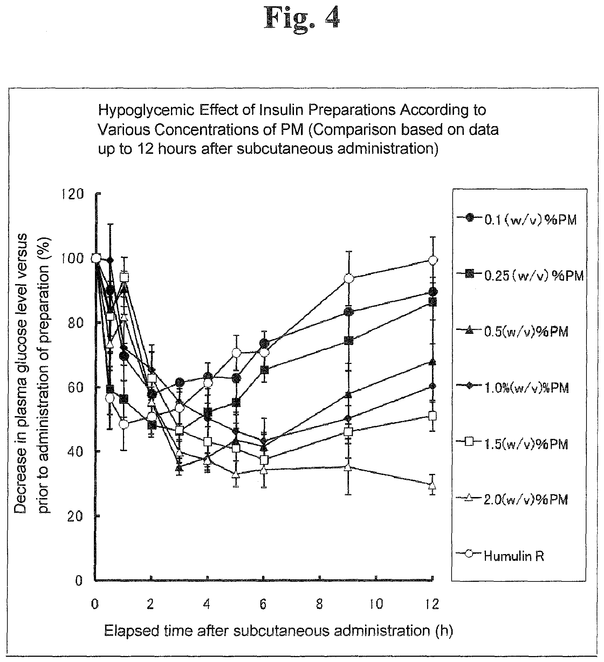

Decreases in plasma glucose concentrations were observed beyond 12 hours in the case of PM concentrations of 0.5% (w/v), 1.0% (w/v), 1.5% (w/v) and 2.0% (w/v) (FIG. 4).

### Study up to 24 Hours after Subcutaneous Administration

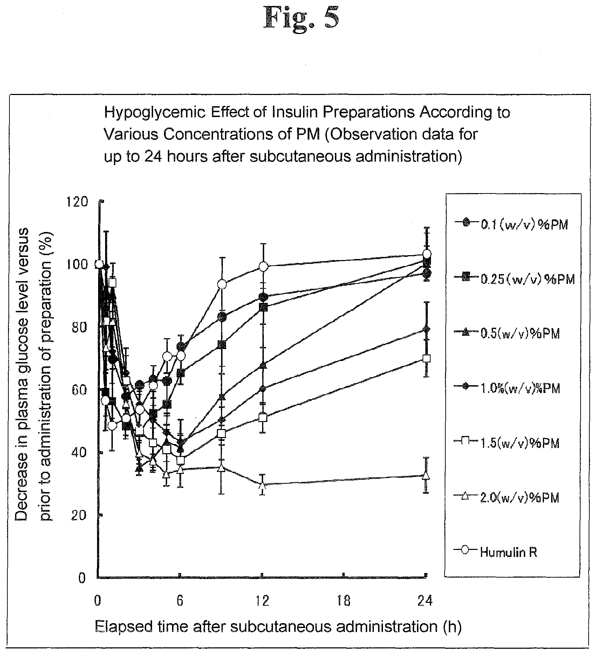

Decreases in plasma glucose concentrations were observed beyond 24 hours in the case of PM concentrations of 1.0% (w/v), 1.5% (w/v) and 2.0% (w/v) (FIG. 5).

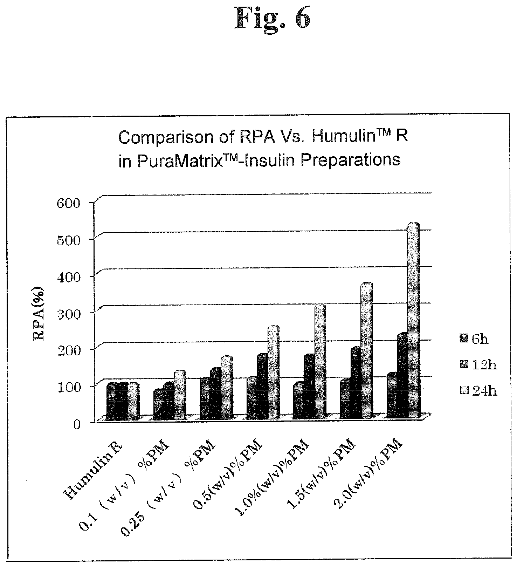

Relative physiological availability (RPA) was determined for Humulin™R. The area above plasma glucose concentrations in the percentage change versus time curves (AAC) was determined for each preparation by dividing into time periods of 6 hours, 12 hours and 24 hours based on data of the changes in plasma glucose concentration, followed by comparison of those results with those of Humulin™R (using average values) (FIG. 6).

It can be seen from the graph that, when examining the results at 24 hours, the effect of 2% PuraMatrix was five times greater than that of Humulin™R and was found to be sustained for a longer period of time.

### INDUSTRIAL APPLICABILITY

The insulin preparation of the present invention is useful as a diabetes therapeutic agent.

## Claims

1. A long-acting insulin preparation for use in a method of treating diabetes, comprising insulin, a self-assembling peptide of SEQ ID NO:1, and a hydrogen and a chloride ion, wherein the concentration of the self-assembling peptide of SEQ ID NO: 1 is 1.0 to 2.0% (w/v).

2. An ultra-fast insulin preparation for use in a method of treating diabetes comprising insulin, a self-assembling peptide of SEQ ID NO: 1, and a hydrogen and chloride ion, wherein the concentration of the self-assembling peptide of SEQ ID NO: 1 is 0.1 to 0.25% (w/v).

3. The insulin preparation according to claim 1 or 2, wherein the insulin is wild-type insulin.

## Patentansprüche

1. Lang wirkendes Insulinpräparat zur Verwendung in einem Verfahren des Behandelns von Diabetes, das Insulin, ein selbstanordnendes Peptid von SEQ ID NO: 1 und ein Wasserstoffion und Chloridion umfasst, wobei die Konzentration des selbstanordnenden Peptids von SEQ ID NO: 1 1,0 bis 2,0 % (w/v) beträgt.

2. Ultraschnelles Insulinpräparat zur Verwendung in einem Verfahren des Behandelns von Diabetes, das Insulin, ein selbstanordnendes Peptid von SEQ ID NO: 1 und ein Wasserstoffion und Chloridion umfasst, wobei die Konzentration des selbstanordnenden Peptids von SEQ ID NO: 1 0,1 bis 0,25 % (w/v) beträgt.

3. Insulinpräparat nach Anspruch 1 oder 2, wobei das Insulin Wildtyp-Insulin ist.

## Revendications

1. Préparation d'insuline à action prolongée destinée à une utilisation dans une méthode de traitement du diabète, comprenant l'insuline, un peptide auto-assemblé de la SEQ ID n° : 1 et un ion hydrogène et chlorure, la concentration du peptide auto-assemblé de la SEQ ID n° : 1 allant de 1,0 à 2,0 % (p/v).

2. Préparation d'insuline à action très rapide destinée à une utilisation dans une méthode de traitement du diabète comprenant l'insuline, un peptide auto-assemblé de la SEQ ID n° : 1, et un ion hydrogène et chlorure, la concentration du peptide auto-assemblé de la SEQ ID n° : 1 allant de 0,1 à 0,25 % (p/v).

3. Préparation d'insuline selon la revendication 1 ou 2, dans laquelle l'insuline est une insuline de type sauvage.
